# EUROPÄISCHE PATENTSCHRIFT

(11) **EP 0 889 038 B1**
(45) Veröffentlichungstag und Bekanntmachung des Hinweises auf die Patenterteilung: **29.05.2002**
(21) Anmeldenummer: 98112192.4
(22) Anmeldetag: 01.07.1998
(51) Int. Cl.: C07D 241/24, C07D 241/20, C07D 241/18

(54) **Verfahren zur Herstellung von Alkoxy- oder Aryloxypyrazinderivaten**
Process for the preparation of alkoxy-or aryloxypyrazine derivatives
Procédé de préparation de dérivés d'alkoxy- ou d'aryloxypyrazines

(30) Priorität: 03.07.1997 CH 161597
(43) Veröffentlichungstag der Anmeldung: 07.01.1999
(73) Patentinhaber: Lonza AG, 3930 Visp (CH)
(72) Erfinder: Fuchs, Rudolf, Dr., 1950 Sion (CH)
(74) Vertreter: Winter, Brandl, Fürniss, Hübner, Röss, Kaiser, Polte Partnerschaft

(56) Entgegenhaltungen:
- FR-A- 1 366 718
- GB-A- 1 269 484
- M.BOTTA ET AL.: "SYNTHESIS OF 2-AMINO-3-METHOXYPYRAZINE." JOURNAL OF HETEROCYCLIC CHEMISTRY., Bd. 16, 1979, Seiten 193-194, XP002081388 PROVO US
- CHEMICAL ABSTRACTS, vol. 88, no. 3, 1978 Columbus, Ohio, US; abstract no. 22973p, Seite 644; Spalte 2; XP002081390 & JP 07 783679 A (YAMANOUCHI)
- HIDEKI HIRANO ET AL.: "THE SUBSTITUTION REACTION OF PYRAZINE-2,3-DICARBONITRILE DERIVATIVES" JOURNAL OF HETEROCYCLIC CHEMISTRY., Bd. 19, 1982, Seiten 1409-1413, XP002081389 PROVO US

## Beschreibung

Die Erfindung betrifft ein neues Verfahren zur Herstellung von Alkoxy- oder Aryloxypyrazinderivaten der allgemeinen Formel 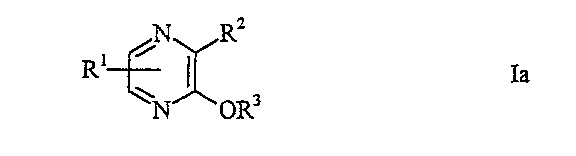 worin R¹ Wasserstoff, gegebenenfalls substituiertes Alkyl oder gegebenenfalls substituiertes Aryl bedeutet, R² für Wasserstoff, gegebenenfalls substituiertes Alkyl, -CONH₂, -COOR⁴, worin R⁴ gegebenenfalls substituiertes Alkyl bedeutet, oder -C(NH)OR⁴, worin R⁴ die genannte Bedeutung hat, steht und R³ gegebenenfalls substituiertes Alkyl oder gegebenenfalls substituiertes Aryl bedeutet.

Ausserdem betrifft die Erfindung ein neues Verfahren zur Herstellung von Alkoxy- oder Aryloxypyrazinaminderivaten der allgemeinen Formel 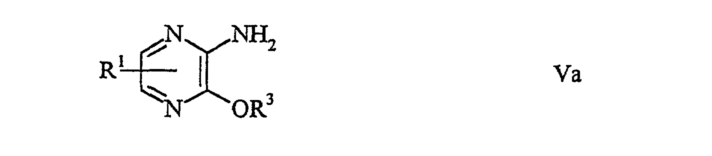 worin R¹ und R³ die genannte Bedeutung haben.

Sowohl die Pyrazinderivate der allgemeinen Formel Ia als auch die Pyrazinaminderivate der allgemeinen Formel Va sind wichtige Zwischenprodukte zur Herstellung von pharmazeutisch aktiven Wirkstoffen (Katritzky, Comprehensive Het. Chem. Vol. 3, 1984, 179 - 197).

Zur Herstellung von Pyrazinderivaten sind aus der o. g. Literaturstelle zahlreiche Verfahren bekannt.

Zum Beispiel beschreibt die GB-A-922 725 ein Verfahren zur Herstellung von 3-Methoxy-5-methylpyrazin-2-amin durch Umsetzung des 3-Chlor-5-methylpyrazin-2-amins mit Natriummethanolat.

Die GB-A-1 269 484 beschreibt die Herstellung von Arylpyrazincarbonsäuren und deren Derivaten ausgehend von Arylglyoxal und Aminomalondiamid.

H. Hirano et al. (J. Heterocyclic Chem, 19:1409, 1982) beschreiben die Herstellung von Arylpyrazincarbonsäurederivaten ausgehend von den entsprechenden Nitrilen.

Die FR-A-1 366 718 beschreibt die Herstellung von Alkoxypyrazinaminderivaten aus den entsprechenden Carbamoylpyrazinderivaten durch Umsetzung mit Alkalihypohalogeniten. M. Botta et al. (J. Heterocyclic Chem, 16:193, 1979) beschreiben die Herstellung von 2-Amino-3-methoxypyrazin durch Umsetzung von 2-Carbamido-3-methoxypyrazin mit Natriumhypochlorit.

Die Aufgabe der Erfindung bestand darin, einen neuen alternativen Zugang zu Alkoxy- oder Aryloxypyrazin- und Alkoxy- oder Aryloxypyrazinaminderivaten zu eröffnen.

Mit den neuen Verfahren gemäss den Ansprüchen 1 und 6 konnte die Aufgabe erfolgreich gelöst werden.

Schlüsselschritt der erfindungsgemässen Synthese gemäss Anspruch 1 ist die Umsetzung eines Glyoxalderivats der allgemeinen Formel 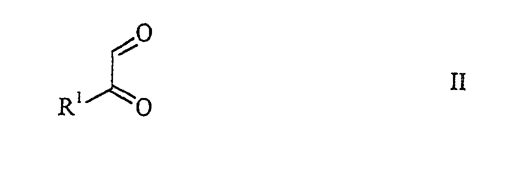 worin R¹ die genannte Bedeutung hat, mit einem Aminoimidat der allgemeinen Formel 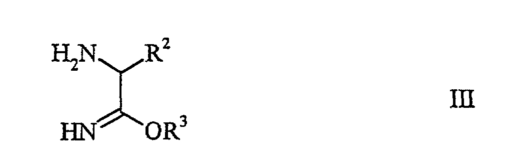 worin R² und R³ die genannte Bedeutung haben, zum Alkoxy- oder Aryloxypyrazinderivat der allgemeinen Formel 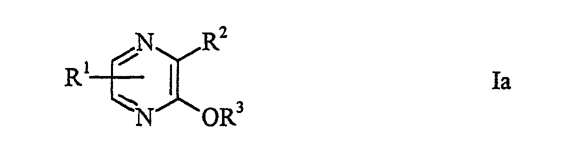 worin R¹ Wasserstoff, gegebenenfalls substituiertes Alkyl oder gegebenenfalls substituiertes Aryl bedeutet, R² für Wasserstoff, gegebenenfalls substituiertes Alkyl, -CONH₂, -COOR⁴, worin R⁴ gegebenenfalls substituiertes Alkyl bedeutet, oder -C(NH)OR⁴, worin R⁴ die genannte Bedeutung hat, steht und R³ gegebenenfalls substituiertes Alkyl oder gegebenenfalls substituiertes Aryl bedeutet.

Die Reste R¹ bis R⁵ haben folgende Bedeutung:

Alkyl hat die Bedeutung einer C₁₋₆-Alkylgruppe; namentlich erwähnt sei Methyl, Ethyl, n-Propyl, i-Propyl, n-Butyl, i-Butyl, t-Butyl, Pentyl und seine Isomeren und Hexyl und seine Isomeren.
Bevorzugt steht Alkyl für eine C₁₋₄-Alkylgruppe. Die Alkylgruppe kann gegebenenfalls substituiert sein mit einer der genannten Alkylgruppen, mit Aryl, mit einem Halogen, mit einer Alkoxygruppe, mit einer Amino-, Alkylamino- oder mit einer Dialkylaminogruppe.

Unter gegebenenfalls substituiertem Aryl wird eine gegebenenfalls substituierte Phenyl- oder Naphthylgruppe verstanden. Substituenten sind die genannten Alkylgruppen, Halogen, Alkoxy, Amino, Alkylamino oder Dialkylamino. Bevorzugte Arylgruppe ist Phenyl. Bevorzugte Aryl-substituierte Alkylgruppe ist Benzyl. Halogen steht für Fluor, Chlor, Brom oder Jod, bevorzugt für Chlor.

Abhängig vom Substitutionsmuster der Reaktanden kann sich die Gruppe R¹ regioselektiv in 5-Stellung oder 6-Stellung des Pyrazinderivates positionieren.

In der Regel und auch bevorzugt resultiert ein Pyrazinderivat der allgemeinen Formel 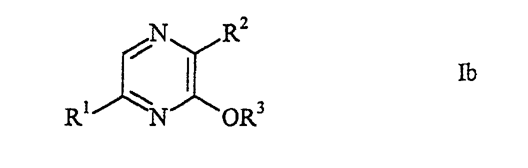 worin R¹ sich in 5-Stellung befindet.

Die Glyoxalderivate der allgemeinen Formel II sind in der Regel kommerziell erhältlich. Dies trifft insbesondere für die bevorzugt eingesetzten Verbindungen Glyoxal (R¹ = H), Methylglyoxal (R¹ = CH₃) und Phenylglyoxal (R¹ = Phenyl) zu. Ebenfalls einsetzbar sind aber noch Glyoxalverbindungen der allgemeinen Formel II mit der Bedeutung von R¹ = t-Butyl oder Halogenalkyl wie z. B. Di- oder Trihalogenmethyl, insbesondere Di- oder Trifluormethyl oder Di- oder Trichlormethyl.

Das Aminoimidat der allgemeinen Formel III als Reaktionspartner des Glyoxalderivats der allgemeinen Formel II ist zwar eine eindeutig nachweisbare, aber in der Regel nicht stabil isolierbare Verbindung. Das Aminoimidat der allgemeinen Formel III wird daher zweckmässig gemäss Anspruch 2 durch Umsetzung eines Aminonitrils der allgemeinen Formel 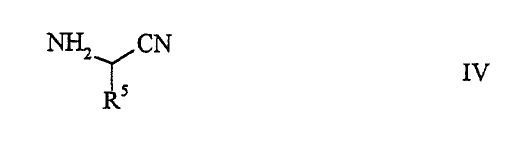 worin R⁵ die Bedeutung von R² oder von Cyano hat, mit einem Alkali- oder Erdalkalialkoholat direkt und ohne Isolation mit dem Glyoxalderivat der allgemeinen Formel II zum Pyrazinderivat der allgemeinen Formel Ia weiterverarbeitet.

Zweckmässig wird so vorgegangen, dass das Aminonitril der allgemeinen Formel IV in einem geeigneten Lösungsmittel, bevorzugt einem aliphatischen Alkohol, vorgelegt wird und mit dem betreffenden Alkali- oder Erdalkalialkoholat bei einer Temperatur von zweckmässig -30 °C bis 150 °C umgesetzt wird.
Bevorzugt gelangen Alkalialkoholate wie zum Beispiel Natrium- oder Kaliummethylat oder -ethylat zur Anwendung.
Abhängig von der Gruppe R² im resultierenden Aminoimidat der allgemeinen Formel III kann es zweckmässig sein, vorgängig das Reaktionsgemisch mit einer geeigneten Säure zu neutralisieren.
Als geeignete Säuren bieten sich einfache Carbonsäuren wie zum Beispiel Essigsäure oder Mineralsäuren wie z. B. Schwefelsäure oder Salzsäure an.

Das Aminoimidat der allgemeinen Formel III ist in der Regel nicht stabil isolierbar, lässt sich aber eindeutig durch spektroskopische Methoden wie ¹³C-NMR nachweisen. Entsprechend schliesst in der Regel direkt die Weiterumsetzung mit dem Glyoxalderivat an. Diese erfolgt zweckmässig bei einer Temperatur von -30 °C bis 150 °C, vorzugsweise von -10 °C bis 10 °C.

Üblicherweise wird das Glyoxalderivat der allgemeinen Formel II in einem leichten Überschuss bezogen auf das Aminonitril der allgemeinen Formel IV eingesetzt.
Die Umsetzung ist in der Regel nach 0,1 h bis 40 h beendet, worauf das Alkoxy- oder Aryloxypyrazin der allgemeinen Formel Ia auf übliche Weise, zum Beispiel durch Extraktion, aus dem Reaktionsgemisch isoliert werden kann.

Pyrazinderivate der allgemeinen Formel Ia', worin R¹ gegebenenfalls substituiertes Alkyl und R³ gegebenenfalls substituiertes Alkyl oder Aryl bedeutet und R² für -CONH₂, -COOR⁴, oder -C(NH)OR⁴, steht, worin R⁴ gegebenenfalls substituiertes Alkyl bedeutet, sind neu und nicht literaturbekannt und sind folglich ebenfalls Gegenstand der vorliegenden Erfindung.

Ebenfalls neu sind die im folgenden namentlich erwähnten Verbindungen
- 3-Methoxy-5-methylpyrazin-2-carbonsäureamid,
- 3-Ethoxy-5-methylpyrazin-2-carbonsäureamid,
- 3-Methoxy-5-methylpyrazin-2-carbonsäuremethylester,
- 3-Ethoxy-5-methylpyrazin-2-carbonsäuremethylester,
- 3-Methoxy-5-phenylpyrazin-2-carbonsäureamid,
- 3-Ethoxy-5-phenylpyrazin-2-carbonsäureamid,
- 3-Methoxy-5-methylpyrazin-2-imidocarbonsäuremethylester,
- 3-Ethoxy-5-methylpyrazin-2-imidocarbonsäuremethylester,
- 3-Methoxy-5-methylpyrazin-2-carbonsäureethylester und
- 3-Ethoxy-5-methylpyrazin-2-carbonsäureethylester.

Bevorzugt eignet sich die erfindungsgemässe Umsetzung zur Herstellung von 3-Methoxy-5-methylpyrazin-2-carbonsäureamid (allgemeine Formel Ib mit R¹ Methyl, R² -CONH₂, R³ Methyl). Dabei kann entweder von 2-Amino-2-cyan-essigsäureamid (allgemeine Formel IV mit R⁵ -CONH₂) oder von 2-Aminomalonsäuredinitril (allgemeine Formel IV mit R⁵ -CN), beziehungsweise einem Salz davon, ausgegangen werden.

Ausgehend von 2-Amino-2-cyan-essigsäureamid erhält man die Zielverbindung nach der zuvor beschriebenen Umsetzung mit dem Alkali- oder Erdalkalialkoholat und anschliessender Neutralisation über das intermediäre Aminoimidat (allgemeine Formel III, R² -CONH₂, R³ Methyl) und nach Umsetzung mit Methylglyoxal (allgemeine Formel II, R¹ Methyl).

Ausgehend von 2-Aminomalonsäuredinitril, beziehungsweise einem Salz davon, erhält man die Zielverbindung nach der zuvor beschriebenen Umsetzung mit dem Alkali- oder Erdalkalialkoholat und anschliessender Neutralisation über das intermediäre Aminoimidat (allgemeine Formel III, R² -C(NH)OCH₃, R³ Methyl), nach dessen Umsetzung mit Methylglyoxal (allgemeinen Formel II, R¹ Methyl) via dem 3-Methoxy-5-methylpyrazin-2-imidocarbonsäure-methylester, nach dessen Ansäuern zum 3-Methoxy-5-methylpyrazin-2-carbonsäuremethylester und schliesslich nach dessen Amidierung.

Bei der letzten Variante wird der 3-Methoxy-5-methylpyrazin-2-imidocarbonsäuremethylester nicht isoliert, sondern durch Ansäuern des Reaktionsgemisches direkt in den genannten Carbonsäureester überführt. Das Ansäuern sowie die Amidierung erfolgt auf bekannte Art und Weise mit einer Mineralsäure, beziehungsweise mit Ammoniak.

Die erfindungsgemäss herstellbaren Alkoxy- oder Aryloxypyrazinderivate der allgemeinen Formel Ia mit der Bedeutung von R² -CONH₂ und R¹ und R³, wie genannt, können, nach einem weiteren Aspekt der Erfindung, nach den Prinzipien des Hofmann Abbaus mit einem Alkalihypohalogenit in Alkoxy- oder Aryloxypyrazinaminderivate der allgemeinen Formel 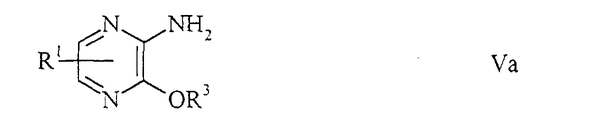 worin R¹ und R³ die genannte Bedeutung haben, überführt werden.

Bevorzugt werden ausgehend von den Alkoxy- oder Aryloxypyrazinderivaten der allgemeinen Formel Ib die Alkoxy- oder Aryloxypyrazinaminderivate der allgemeinen Formel 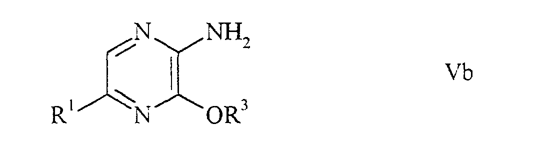 worin R¹ und R³ die genannte Bedeutung haben, hergestellt.

Die Hofmann Abbau Reaktion ist literaturbekannt. In der Regel erfolgt die Umsetzung mit einer Alkalihypobromit-Lösung, welche üblicherweise aus dem entsprechenden Alkalihydroxid und Brom zubereitet wird, bei einer Reaktionstemperatur zwischen -20 °C und 100°C.
Das Alkoxy- oder Aryloxypyrazinaminderivat kann auf fachmännisch übliche Weise, zum Beispiel durch Extraktion, aus dem Reaktionsgemisch isoliert werden.

### Beispiele

### Beispiel 1

### a) Synthese von (2-Amino-2-carbamoyl) essigimidsäuremethylester

1,09 g (10,3 mmol) 2-Amino-2-cyanessigsäureamid wurden in 11 g Methanol unter Argon vorgelegt. Man gab 0,29 g (1,6 mmol) Natriummethanolat-Lösung (30%) zu und rührte 2 bei 20 °C Die Struktur des Titelproduktes wurde mittels NMR-Messung bestätigt..
- ¹H-NMR (DMSO-d₆, 400 MHz):: 3,85 (s, 3H),
4,18 (s, 1H);
8,2 - 8,6 (sb, 1H).
- ¹³C-NMR (DMSO-d₆, 400 MHz):: 52,27 (q);
55,83 (d);
171,66 (s);
172,78 (s).

### b) Synthese von 3-Methoxy-5-methylpyrazin-2-carbonsäureamid

6 g (60,5 mmol) 2-Amino-2-cyanessigsäureamid wurden in 67 g Methanol unter Argon vorgelegt. Man gab 1,67 g (9,3 mmol) Natriummethanolat-Lösung (30 %) zu und rührte 2 Stunden bei 20 °C. Nach Neutralisation mit 0,558 g (9,3 mmol) Essigsäure gab man 11,55 g (64,1 mmol) Methylglyoxal-Lösung (40 %) zu. Man rührte 2 Stunden bei 20 °C, dann 2 Stunden bei 50 °C. Das Lösungsmittel wurde abdestilliert und das 3-Methoxy-5-methylpyrazin-2-carbonsäureamid durch Säulenchromatographie (Fliessmittel: Essigsäureethylester / Methanol, 4 : 1) gereinigt. Man erhielt 5 g 3-Methoxy-5-methylpyrazin-2-carbonsäureamid.
(Ausbeute: 50 %)
- ¹H-NMR (DMSO-d₆, 400 MHz):: 8,10 (s, 1H);
7,84 und 7,56 (2s, breit 2H);
3,93 (s, 3H);
2,46 (s, 3H).
- ¹³C-NMR (DMSO-d₆, 400 MHz):: 165,5 (s);
156,6(s);
152,4 (s);
134,5 (s);
134,3 (d);
53,4 (q);
20,7 (q).

### c) Synthese von 3-Methoxy-5-methylpyrazin-2-carbonsäureamid

6 g (60,5 mmol) 2-Amino-2-cyanessigsäureamid wurden in 67 g Methanol unter Argon vorgelegt. Man gab 1,67 g (9,3 mmol) Natriummethanolat-Lösung (30%) zu und rührte 2 h bei 20 °C. Bei 0 °C gab man 11,55 g (64,1 mmol) Methylglyoxal-Lösung (40%) zu und rührte 2 h bei 0 °C. Dann wurde die Lösung auf -20 °C abgekühlt. Das Produkt fiel aus. Nach der Filtration und Trocknung erhielt man 3,56 des Titelproduktes.
(Ausbeute: 39%)
- ¹H-NMR (DMSO-d₆, 400 MHz) :: 8,10 (s, 1H);
7,84 und 7,56 (2s breit, 2H);
3,93 (s, 3H);
2,46 (s, 3H).
- ¹³C-NMR (DMSO-d₆, 400 MHz):: 165,5 (s);
156,6 (s),
152,4 (s);
134,5 (s);
134,3 (d);
53,4 (q);
20,7 (q).
- Schmelzpunkt:: 170 - 172 °C

### Beispiel 2

### a) Synthese von 3-Methoxy-5-methylpyrazin-2-amin

3,71 g (56,2 mmol) Kaliumhydroxid (85 %) und 31 g Wasser wurden in einem Kolben vorgelegt. Bei 1 °C tropfte man 2,16 g (13,5 mmol) Brom während 10 Minuten hinzu. Diese Kaliumhypobromit-Lösung wurde zu einer wässrigen Lösung von 2,27 g (13,1 mmol) 3-Methoxy-5-methylpyrazin-2-carbonsäureamid in 12 g Wasser bei 4 °C zugetropft. Man rührte eine Stunde bei 1 °C, dann 3 Stunden bei 98 °C. Das erhaltene 3-Methoxy-5-methylpyrazin-2-amin wurde bei 20 °C mit Methylenchlorid (zweimal 25 ml) extrahiert. Nach Entfernung des Lösungsmittels erhielt man 0,92 g 3-Methoxy-5-methylpyrazin-2-amin.
(Ausbeute: 50,4 %)
- ¹H-NMR (DMSO-d₆, 400 MHz):: 2,20 (s, 3H);
3,87 (s, 3H);
5,90 (s, 2H);
7,33 (s, 1H).
- Schmelzpunkt:: 75 °C - 76,5 °C

### b) Synthese von 3-Methoxy-5-methylpyrazin-2-amin

1,54 g (23,3 mmol) Kaliumhydroxid (85%) und 15 g Wasser wurden in einem Kolben vorgelegt. Bei 1 °C tropfte man 1,08 g (5,53 mmol) Brom während 10 Minuten hinzu. Diese Kaliumhypobromit-Lösung wurde zu einer wässrigen Lösung von 1,04 g (5,76 mmol) 3-Methoxy-5-methylpyrazin-2-carbonsäureamid, in 6,5 g Wasser bei 4 °C zugetropft. Man rührte 1 h bei 1 °C, dann 3 h bei 83 °C Das 3-Methoxy-5-methylpyrazin-2-amin wurde bei 20 °C mit Methylenchlorid (zweimal 15 ml) extrahiert. Nach Entfernung des Lösungsmittels erhielt man 0,65 g des Titelproduktes.
(Ausbeute: 80%)
- ¹H-NMR (DMSO-d₆, 400 MHz) :: 2,20 (s, 3H);
3,87 (s, 3H);
5,90 (s, 2H);
7,33 (s, 1H).
- Schmelzpunkt:: 75 - 76,5 °C.

### Beispiel 3

### Synthese von 3-Methoxypyrazin-2-carbonsäureamid

1 g (10,1 mmol) 2-Amino-2-cyanessigsäureamid wurden in 10 g Methanol unter Argon vorgelegt. Man gab 0,25 g (1,4 mmol) Natriummethanolat-Lösung (30 %) zu und rührte 2 Stunden bei 20 °C. Nach Neutralisation mit 0,084 g (1,4 mmol) Essigsäure gab man 2,27 g (20 mmol) Glyoxal-Lösung (40 %) zu. Man rührte 2 Stunden bei 20 °C, dann 2 Stunden bei 50 °C. Das Lösungsmittel wurde abdestilliert. Man erhielt 0,75 g 3-Methoxypyrazin-2-carbonsäureamid.
(Ausbeute: 50 %)
- ¹H-NMR (DMSO-d₆, 400 MHz):: 3,18 (s, 3H);
3,95 (s, 3H);
7,63 (s, 1H);
7,93 (s, 1H);
8,22 (d, 1H, J = 1Hz);
8,37 (d, 1H, J = 1Hz).

### Beispiel 4

### Synthese von 3-Methoxy-5-methylpyrazin-2-carbonsäuremethylester

5 g (19,3 mmol) 2-Aminomalonsäuredinitril-4-toluolsulfonat wurden in 50 g Methanol unter Argon vorgelegt. Man gab 4,09 g (22,7 mmol) Natriummethanolat-Lösung (30 %) zu und rührte 2 Stunden bei 2 °C, dann 2 Stunden bei 20 °C. Nach Neutralisation mit 0,204 g (3,4 mmol) Essigsäure gab man 3,6 g (19,9 mmol) Methylglyoxal-Lösung (40 %) zu. Man rührte 2 Stunden bei 40 °C, dann, bei 20 °C, gab man 9,2 g (80 mmol) Salzsäure (32 %) zu und rührte 6 Stunden bei 20 °C. Das Lösungsmittel wurde abdestilliert und das 3-Methoxy-5-methylpyrazin-2-carbonsäuremethylester mit Methylenchlorid extrahiert.
Man erhielt 1 g 3-Methoxy-5-methylpyrazin-2-carbonsäuremethylester.
(Ausbeute: 27 %).
- ¹H-NMR (CDCl₃, 400 MHz):: 2,54 (s, 3H);
3,98 (s, 3H);
4,07 (s, 1H);
8,12 (s, 1H).
- ¹³C-NMR (CDCl₃, 400 MHz):: 21,4 (q);
52,7 (q);
54,2 (q);
129,7 (s);
135,4 (d);
155,4 (s);
158,9 (s);
164,3 (s).

### Beispiel 5

### Synthese von 3-Methoxy-5-methylpyrazin-2-carbonsäureamid

1 g (5,5 mmol) 3-Methoxy-5-methylpyrazin-2-carbonsäuremethylester wurden in 15 ml (198 mmol) NH₃ (25 %) vorgelegt und bei 50 °C gerührt. Nach Einengen erhielt man 0,8 g 3-Methoxy-5-methylpyrazin-2-carbonsäureamid.
(Ausbeute 86%)
- ¹H-NMR (DMSO-d₆, 400 MHz) :: 8,10 (s,1H);
7,84 und 7,56 (2s breit,2H);
3,93 (s,3H);
2,46 (s,3H).

### Beispiel 6

### Synthese von 3-Methoxy-5-phenylpyrazin-2-carbonsäureamid

2 g (20 mmol) 2-Amino-2-cyanessigsäureamid wurden in 15 g Methanol unter Argon vorgelegt. Man gab 0,55 g (3 mmol) Natriummethanolat-Lösung (30%) zu und rührte 2 h bei 20 °C. Bei 0 °C gab man 3,2 g (21 mmol) Phenylglyoxal zu, rührte 2 bei 0 °C und engte ein.
Das Produkt wurde durch Säulenchromatographie gereinigt (Fliessmittel Essigsäureethylester / Methanol: 4/1).
Man erhielt 3 g 3-Methoxy-5-Phenylpyrazin-2-carbonsäureamid.
(Ausbeute 65 %).
- ¹H-NMR (DMSO-d₆, 400 MHz) :: 4,04 (s,3H);
7,5-8,0 (m,7H);
8,83 (s,1H)
- ¹³C-NMR (DMSO-d₆, 400 MHz) :: 53,45;
127;
128,77;
130,24;
131,7;
134,99;
135,90;
149,4;
156,66;
165,3.
- MS:: 229 (100%)

## Patentansprüche

1. Verfahren zur Herstellung von Alkoxy- oder Aryloxypyrazinderivaten der allgemeinen Formel 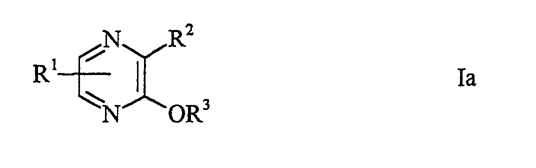 worin R¹ Wasserstoff, gegebenenfalls mit Halogen, Alkyl, Alkoxy, Aryl, Amino, Alkylamino oder Dialkylamino substituiertes Alkyl oder gegebenenfalls mit Halogen, Alkyl, Alkoxy, Amino, Alkylamino oder Dialkylamino substituiertes Aryl bedeutet, R² für Wasserstoff, gegebenenfalls wie oben substituiertes Alkyl, -CONH₂, -COOR⁴, worin R⁴ gegebenenfalls wie oben substituiertes Alkyl bedeutet, oder -C(NH)OR⁴, worin R⁴ die genannte Bedeutung hat, steht und R³ gegebenenfalls wie oben substituiertes Alkyl oder Aryl bedeutet, **dadurch gekennzeichnet, dass** ein Glyoxalderivat der allgemeinen Formel 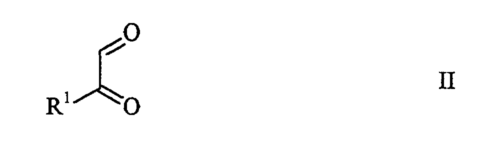 worin R¹ die genannte Bedeutung hat, mit einem Aminoimidat der allgemeinen Formel 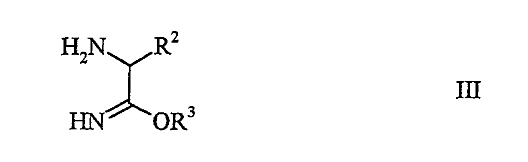 worin R² und R³ die genannten Bedeutung haben, umgesetzt wird.

2. Verfahren nach Anspruch 1, **dadurch gekennzeichnet, dass** das Aminoimidat der allgemeinen Formel III durch Umsetzung eines Aminonitrils der allgemeinen Formel 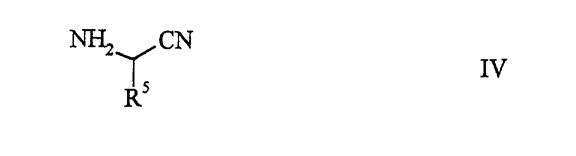 worin R⁵ die Bedeutung von R² oder von Cyano hat, mit einem Alkali- oder Erdalkalialkoholat hergestellt wird und danach direkt ohne Isolierung mit dem Glyoxalderivat der allgemeinen Formel II zum Alkoxy- oder Aryloxypyrazinderivat der allgemeinen Formel Ia weiterumgesetzt wird.

3. Verfahren nach einem der Ansprüche 1 oder 2 zur Herstellung von 3-Methoxy-5-methylpyrazin-2-carbonsäureamid, **dadurch gekennzeichnet, dass** 2-Amino-2-cyanessigsäureamid mit einem Alkali- oder Erdalkalialkoholat und anschliessender Neutralisation in das Aminoimidat der allgemeinen Formel III, worin R² -CONH₂ und R³ Methyl bedeutet, überführt wird und dieses, ohne Isolierung, mit Methylglyoxal umgesetzt wird.

4. Verfahren nach einem der Ansprüche 1 oder 2 zur Herstellung von 3-Methoxy-5-methylpyrazin-2-carbonsäureamid, **dadurch gekennzeichnet, dass** 2-Aminomalonsäuredinitril, beziehungsweise ein Salz davon, mit einem Alkali- oder Erdalkalialkoholat und anschliessender Neutralisation in das Aminoimidat der allgemeinen Formel III, worin R² -C(NH)OCH₃ und R³ Methyl bedeutet, überführt wird, dieses, ohne Isolierung, mit Methylglyoxal zur Umsetzung gebracht wird, nach Ansäuern zunächst in den 3-Methoxy-5-methylpyrazin-2-carbonsäuremethylester überführt wird und schliesslich die Esterfunktion amidiert wird.

5. Verfahren nach einem der Ansprüche 1 bis 4, **dadurch gekennzeichnet, dass** die Umsetzung mit einem Alkali- oder Erdalkalialkoholat sowie die Umsetzung mit dem Glyoxalderivat bei einer Temperatur zwischen -30 °C und 150 °C durchgeführt wird.

6. Verfahren nach einem der Ansprüche 1 bis 5, **dadurch gekennzeichnet, dass** ein Alkoxy- oder Aryloxypyrazinderivat der allgemeinen Formel Ia, worin R² -CONH₂ bedeutet, mit einem Alkalihypohalogenit zu einem Alkoxy- oder Aryloxypyrazinaminderivat der allgemeinen Formel 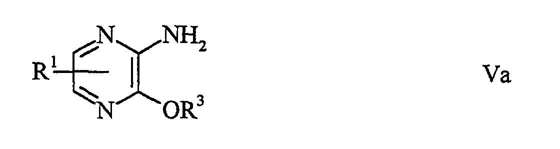 worin R¹ und R³ die genannte Bedeutung haben, weiterumgesetzt wird.

7. Alkoxy- oder Aryloxypyrazinderivate der allgemeinen Formel 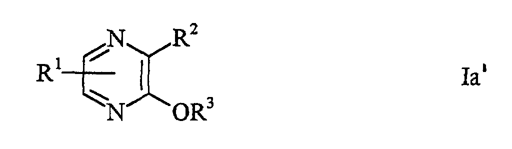 worin R¹ gegebenenfalls mit Halogen, Alkyl, Alkoxy, Aryl, Amino, Alkylamino oder Dialkylamino substituiertes Alkyl und R³ gegebenenfalls mit Halogen, Alkyl, Alkoxy, Aryl, Amino, Alkylamino oder Dialkylamino substituiertes Akyl oder gegebenenfalls mit Halogen, Alkyl, Alkoxy, Amino, Alkylamino oder Dialkylamino substituiertes Aryl bedeutet und R² CONH₂, COOR⁴ oder C(NH)OR⁴ bedeutet, worin R⁴ für gegebenenfalls wie oben substituiertes Alkyl steht.

8. Alkoxypyrazinderivat nach Anspruch 7, ausgewählt an der Reihe
3-Methoxy-5-methylpyrazin-2-carbonsäureamid
3-Ethoxy-5-methylpyrazin-2-carbonsäureamid
3-Methoxy-5-methylpyrazin-2-carbonsäuremethylester
3-Ethoxy-5-methylpyrazin-2-carbonsäuremethylester
3-Methoxy-5-methylpyrazin-2-imidocarbonsäuremethylester
3-Ethoxy-5-methylpyrazin-2-imidocarbonsäuremethylester
3-Methoxy-5-methylpyrazin-2-carbonsäureethylester
3-Ethoxy-5-methylpyrazin-2-carbonsäureethylester

9. Alkoxypyrazinderivat, ausgewählt aus der Reihe
3-Methoxy-5-phenylpyrazin-2-carbonsäureamid
3-Ethoxy-5-phenylpyrazin-2-carbonsäureamid

## Claims

1. A process for the production of alkoxy- or aryloxypyrazine derivatives of the general formula 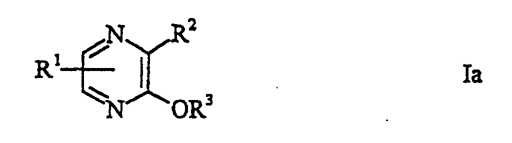 in which R¹ means hydrogen, alkyl optionally substituted with halogen, alkyl, alkoxy, aryl, amino, alkylamino or dialkylamino, or aryl optionally substituted with halogen, alkyl, alkoxy, amino, alkylamino or dialkylamino, R² denotes hydrogen, alkyl optionally substituted as above, -CONH₂, -COOR⁴, in which R⁴ means alkyl optionally substituted as above, or -C(NH)OR⁴, in which R⁴ has the stated meaning, and R³ means alkyl or aryl optionally substituted as above, **characterised in that** a glyoxal derivative of the general formula 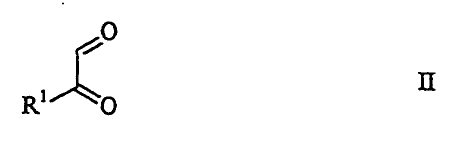 in which R¹ has the stated meaning, is reacted with an aminoimidate of the general formula 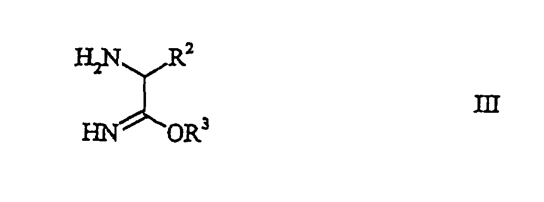 in which R² and R³ have the stated meaning.

2. A process according to claim 1, **characterised in that** the aminoimidate of the general formula III is produced by reacting an aminonitrile of the general formula 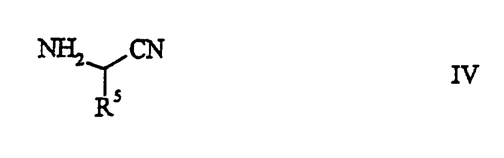 in which R⁵ has the meaning of R² or of cyano, with an alkoxide of an alkali metal or alkaline earth metal and, without being isolated, is thereupon directly further reacted with the glyoxal derivative of the general formula II to yield the alkoxy- or aryloxypyrazine derivative of the general formula Ia.

3. A process according to one of claims 1 or 2 for the production of 3-methoxy-5-methylpyrazine-2-carboxylic acid amide, **characterised in that** 2-amino-2-cyanoacetamide is transformed with an alkoxide of an alkali metal or alkaline earth metal and subsequent neutralisation into the aminoimidate of the general formula III, in which R² means -CONH₂ and R³ means methyl and, without being isolated, said aminoimidate is reacted with methylglyoxal.

4. A process according to one of claims 1 or 2 for the production of 3-methoxy-5-methylpyrazine-2-carboxylic acid amide, **characterised in that** 2-aminomalonic acid dinitrile, or a salt thereof, is transformed with an alkoxide of an alkali metal or alkaline earth metal and subsequent neutralisation into the aminoimidate of the general formula III, in which R² means -C(NH)OCH₃ and R³ means methyl, said aminoimidate, without being isolated, is reacted with methylglyoxal, is transformed, once acidified, initially into the 3-methoxy-5-methylpyrazine-2-carboxylic acid methyl ester and, finally, the ester function is amidated.

5. A process according to one of claims 1 to 4, **characterised in that** the reaction with an alkoxide of an alkali metal or alkaline earth metal and the reaction with the glyoxal derivative is performed at a temperature between -30°C and 150°C.

6. A process according to one of claims 1 to 5, **characterised in that** an alkoxy- or aryloxypyrazine derivative of the general formula Ia, in which R² means -CONH₂, is further reacted with an alkali metal hypohalite to yield an alkoxy- or aryloxypyrazine amine derivative of the general formula 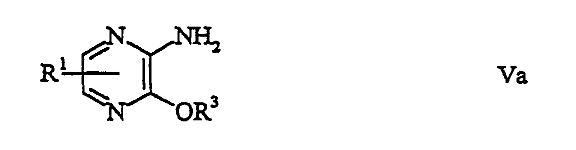 in which R¹ and R³ have the stated meaning.

7. Alkoxy- or aryloxypyrazine derivatives of the general formula 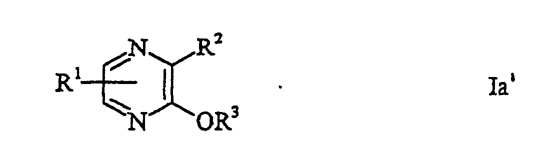 in which R¹ means alkyl optionally substituted with halogen, alkyl, alkoxy, aryl, amino, alkylamino or dialkylamino and R³ means alkyl optionally substituted with halogen, alkyl, alkoxy, aryl, amino, alkylamino or dialkylamino, or aryl optionally substituted with halogen, alkyl, alkoxy, amino, alkylamino or dialkylamino and R² means CONH₂, COOR⁴ or C(NH)OR⁴, in which R⁴ denotes alkyl optionally substituted as above.

8. An alkoxypyrazine derivative according to claim 7, selected from the range
3-methoxy-5-methylpyrazine-2-carboxylic acid amide
3-ethoxy-5-methylpyrazine-2-carboxylic acid amide
3-methoxy-5-methylpyrazine-2-carboxylic acid methyl ester
3-ethoxy-5-methylpyrazine-2-carboxylic acid methyl ester
3-methoxy-5-methylpyrazine-2-imidocarboxylic acid methyl ester
3-ethoxy-5-methylpyrazine-2-imidocarboxylic acid methyl ester
3-methoxy-5-methylpyrazine-2-carboxylic acid ethyl ester
3-ethoxy-5-methylpyrazine-2-carboxylic acid ethyl ester.

9. An alkoxypyrazine derivative selected from the range
3-methoxy-5-phenylpyrazine-2-carboxylic acid amide
3-ethoxy-5-phenylpyrazine-2-carboxylic acid amide.

## Revendications

1. Procédé de préparation de dérivés d'alcoxy- ou aryloxypyrazine de formule générale 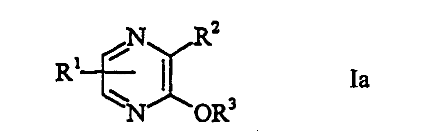 dans laquelle R¹ représente un atome d'hydrogène, un groupe alkyle éventuellement substitué par halogéno, alkyle, alcoxy, aryle, amino, alkylamino ou dialkylamino ou un groupe aryle éventuellement substitué par halogéno, alkyle, alcoxy, amino, alkylamino ou dialkylamino, R² représente un atome d'hydrogène, un groupe alkyle éventuellement substitué de la manière indiquée ci-dessus, un groupe -CONH₂, un groupe -COOR⁴ dans lequel R⁴ représente un groupe alkyle éventuellement substitué de la manière indiquée ci-dessus, ou un groupe -C(NH)OR⁴ dans lequel R⁴ a la signification indiquée, et R³ représente un groupe alkyle ou aryle éventuellement substitué de la manière indiquée ci-dessus, **caractérisé en ce que** l'on fait réagir un dérivé de glyoxal de formule générale 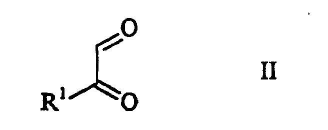 dans laquelle R¹ a la signification indiquée, avec un aminoimidate de formule générale 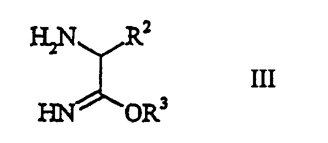 dans laquelle R² et R³ ont la signification indiquée.

2. Procédé selon la revendication 1, **caractérisé en ce que** l'on prépare l'aminoimidate de formule générale III par réaction d'un aminonitrile de formule générale 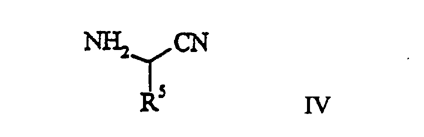 dans laquelle R⁵ a la signification de R² ou représente un groupe cyano, avec un alkylate de métal alcalin ou de métal alcalino-terreux, puis on le fait réagir directement, sans l'isoler, avec le dérivé de glyoxal de formule générale II pour obtenir le dérivé d'alcoxy- ou d'aryloxypyrazine de formule générale Ia.

3. Procédé selon l'une des revendications 1 ou 2 pour la préparation du 3-méthoxy-5-méthylpyrazine-2-carboxamide, **caractérisé en ce que** l'on transforme du 2-amino-2-cyanoacétamide par réaction avec un alkylate de métal alcalin ou de métal alcalino-terreux, puis neutralisation, en l'aminoimidate de formule générale III dans laquelle R² représente le groupe -CONH₂ et R³ le groupe méthyle, et on fait réagir ce dernier, sans l'isoler, avec du méthylglyoxal.

4. Procédé selon l'une des revendications 1 ou 2 pour la préparation du 3-méthoxy-5-méthylpyrazine-2-carboxamide, **caractérisé en ce que** l'on transforme du 2-aminomalodinitrile, ou un de ses sels, par réaction avec un alkylate de métal alcalin ou de métal alcalino-terreux, puis neutralisation, en l'aminoimidate de formule générale III dans laquelle R² représente le groupe -C(NH)OCH₃ et R³ le groupe méthyle, on fait réagir ce dernier, sans l'isoler, avec du méthylglyoxal, on le transforme d'abord, après acidification, en le 3-méthoxy-5-méthylpyrazine-2-carboxylate de méthyle, et on amide finalement la fonction ester.

5. Procédé selon l'une des revendications 1 à 4, **caractérisé en ce que** l'on effectue la réaction avec un alkylate de métal alcalin ou de métal alcalino-terreux et la réaction avec le dérivé de glyoxal à une température comprise entre -30°C et 150°C.

6. Procédé selon l'une des revendications 1 à 5, **caractérisé en ce que** l'on fait ensuite réagir un dérivé d'alcoxy- ou aryloxypyrazine de formule générale Ia dans laquelle R² représente un groupe -CONH₂ avec un hypohalogénite de métal alcalin pour obtenir un dérivé d'alcoxy- ou aryloxypyrazinamine de formule générale 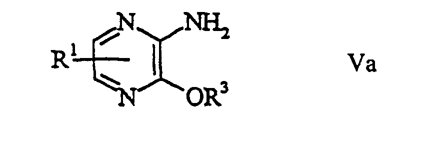 dans laquelle R¹ et R³ ont la signification indiquée.

7. Dérivés d'alcoxy- ou aryloxypyrazine de formule générale 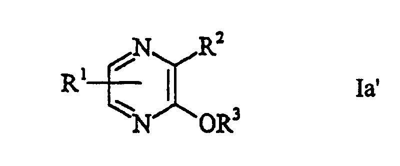 dans laquelle R¹ représente un groupe alkyle éventuellement substitué par halogéno, alkyle, alcoxy, aryle, amino, alkylamino ou dialkylamino, R³ représente un groupe alkyle éventuellement substitué par halogéno, alkyle, alcoxy, aryle, amino, alkylamino ou dialkylamino ou un groupe aryle éventuellement substitué par halogéno, alkyle, alcoxy, amino, alkylamino ou dialkylamino, et R² représente un groupe CONH₂, COOR⁴ ou C(NH)OR⁴, où R⁴ représente un groupe alkyle éventuellement substitué de la manière indiquée ci-dessus.

8. Dérivé d'alcoxypyrazine selon la revendication 7, choisi dans la série constituée par
le 3-méthoxy-5-méthylpyrazine-2-carboxamide,
le 3-éthoxy-5-méthylpyrazine-2-carboxamide,
le 3-méthoxy-5-méthylpyrazine-2-carboxylate de méthyle,
le 3-éthoxy-5-méthylpyrazine-2-carboxylate de méthyle,
le 3-méthoxy-5-méthylpyrazine-2-imidocarboxylate de méthyle,
le 3-éthoxy-5-méthylpyrazine-2-imidocarboxylate de méthyle,
le 3-méthoxy-5-méthylpyrazine-2-carboxylate d'éthyle,
le 3-éthoxy-5-méthylpyrazine-2-carboxylate d'éthyle.

9. Dérivé d'alcoxypyrazine choisi dans la série constituée par
le 3-méthoxy-5-phénylpyrazine-2-carboxamide,
le 3-éthoxy-5-phénylpyrazine-2-carboxamide.
